# EUROPÄISCHE PATENTSCHRIFT

(11) **EP 1 830 707 B1**
(45) Veröffentlichungstag und Bekanntmachung des Hinweises auf die Patenterteilung: **11.05.2011**
(21) Anmeldenummer: 05824859.2
(22) Anmeldetag: 10.12.2005
(51) Int. Cl.: A61B 5/15

(54) **VERFAHREN ZUR HERSTELLUNG EINES STECHELEMENTS**
METHOD FOR PRODUCING A PRICKING ELEMENT
PROCEDE DE PRODUCTION D'UN ELEMENT DE PIQUAGE

(30) Priorität: 17.12.2004 EP 04029926
(43) Veröffentlichungstag der Anmeldung: 12.09.2007
(73) Patentinhaber: F. Hoffmann-La Roche AG, 4070 Basel (CH); Roche Diagnostics GmbH, 68305 Mannheim (DE)
(72) Erfinder: GRISS, Patrick, 8112 Otelfingen (CH); ZIPFEL, Marzellinus, 79112 Freiburg (DE); LOPEZ, Angel, 75180 Pforzheim (DE)
(74) Vertreter: Pfiz, Thomas
(86) Internationale Anmeldenummer: PCT/EP2005/013266
(87) Internationale Veröffentlichungsnummer: WO 2006/066744

(56) Entgegenhaltungen:
- EP-A- 0 445 820
- WO-A-2004/008203
- US-A- 4 777 096
- US-B1- 6 406 638

## Beschreibung

Die Erfindung betrifft ein Verfahren zur Herstellung eines Stechelements insbesondere zur Entnahme einer Körperflüssigkeit, bei welchem aus einem Flachmaterial unter Aufbringen einer Maske und Einwirkung eines Ätzmittels ein Flachformteil gebildet wird, wobei mindestens eine Partie des Flachformteils als Spitze zum Einstechen in ein Körperteil eines Probanden ausgeformt wird.

Stechelemente dieser Art erlauben speziell in der Bioanalytik die Untersuchung geringster Fluidmengen, wie sie beispielsweise für Blutglucosebestimmungen in situ als Kapillarblut entnommen werden. Solche MikrofluidikSysteme zeichnet sich dabei neben den mikroskopischen Volumina (Mikroliter und weniger) auch durch immer kleiner dimensionierte Strukturelemente aus, die eine Ausnutzung von Kapillarkräften erlauben und in so genannten Disposibles für eine Massenfertigung geeignet realisiert werden. Aus dem Bereich der Halbleitertechnologie sind zwar Fertigungsverfahren speziell in Form des Maskenätzens ("photochemical etching") für hochintegrierte Systeme bekannt; die dort verwendeten Materialien lassen sich aber vor allem aufgrund ihrer Sprödigkeit für mechanisch beanspruchte Strukturen kaum einsetzen. Beim Ätzen von biokompatiblen Materialien wie Stahl tritt mit herkömmlichen formkomplementären Ätzmasken das Problem auf, dass die erzeugten Stechstrukturen abgerundet werden und somit keinen besonders optimalen Einstich erlauben.

Aus der US 4 777 096 A ist ein Ätzverfahren zur Herstellung chirurgischer Nadeln bekannt, bei welchem die Ätzmaske sich in einem distal abgestumpften Formbereich über die zu bildende Spitze hinaus erstreckt, womit eine Abrundung vermieden werden soll. Dieser Maskenüberstand ist aber kürzer als die Unterätzreichweite des Ätzmittels, so dass die Spitze durch eine gemeinsame laterale und frontale Ätzwirkung geformt wird, wodurch sich das Problem der Abstumpfung nur graduell reduziert.

Ausgehend hiervon liegt der Erfindung die Aufgabe zugrunde, die im Stand der Technik aufgetretenen Nachteile zu vermeiden und ein Herstellungsverfahren der eingangs genannten Art dahingehend zu verbessern, dass scharfe Stechstrukturen für einen optimierten Einstich in eine Körperpartie in einem für die Massenfertigung geeigneten Prozessablauf geschaffen werden, ohne dass aufwändige Nachbearbeitungsschritte erforderlich wären.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine scharfe Spitze allein durch seitlich aufeinander zulaufende Ätzmittelfronten zu schaffen. Dementsprechend wird erfindungsgemäß vorgeschlagen, dass die Maske mit einem Formbereich für die zu bildende Spitze und einem distal darüber hinaus sich erstreckenden Abschirmbereich gegen frontales Abätzen der Spitze versehen wird, und dass durch laterales Unterätzen der Seitenflanken des Formund Abschirmbereichs eine scharfe Spitze freigelegt wird. Dabei können Konturabrundungen im Bereich der Spitze vermieden werden, wobei der Ätzprozess beendet wird, sobald die zwischen den Seitenflanken der Ätzmaske liegende Materialbrücke durchbrochen ist. Auf diese Weise lassen sich optimierte Spitzen schaffen, um den Einstichschmerz gering zu halten und mit möglichst kleinen Dimensionen Körperflüssigkeit aufnehmen zu können. Zudem lassen sich die erforderlichen Vorschubkräfte für den Einstich minimieren, und eine Traumatisierung des Körpergewebes kann weitgehend vermieden werden.

Vorteilhafterweise verengt sich der der Formbereich der Ätzmaske zu dem Abschirmbereich hin, so dass ein auf die Spitze zulaufender Stechschaft erzeugt wird. Hierfür ist es günstig, wenn der Formbereich zumindest einseitig linear abgeschrägt ist.

Eine weitere vorteilhafte Ausführung sieht vor, dass zumindest eine Seitenflanke im Übergang zwischen Formbereich und Abschirmbereich abgewinkelt oder konkav gekrümmt ist, so dass das Substratmaterial im Bereich der zu erzeugenden Spitze nur von der Seite her abgetragen wird.

In jedem Fall sollte gewährleistet sein, dass der Abschirmbereich über eine gegebene Abschirmlänge im Anschluss an den Formbereich eine gleich bleibende oder zunehmende Breite aufweist. Dabei sollte die Abschirmlänge größer als die proximale Unterätzweite aufgrund der Einwirkung des Ätzmittels sein. Dadurch wird ein zentrales Abätzen gegen die Spitze zuverlässig verhindert.

Bevorzugt wird die Spitze aufgrund einer Engstelle der Maske geformt, wobei die Breite der Engstelle kleiner als die zweifache laterale Unterätzweite sein sollte, so dass das unter der Engstelle liegende Substratmaterial vollständig abgeätzt wird.

Vorteilhafterweise wird der Abschirmbereich im Anschluss an die Engstelle wieder verbreitert. Auch um die Stabilität der Ätzmaske zu verbessern, ist es von Vorteil, wenn der Abschirmbereich einen in distaler Richtung der zu bildenden Spitze gesehen sich erweiternden Brückenabschnitt aufweist.

Während Ätzprozesse in Stahl überwiegend isotrop ablaufen, ist es auch denkbar, dass die laterale Unterätzweite größer oder kleiner als die Ätztiefe ist. Eine Anisotropie tritt zum Beispiel dann auf, wenn das Ätzmittel unter Überdruck gegenüber der Umgebung angewandt wird, um eine besonders effektive Einwirkung sicherzustellen. Das Ätzmittel kann im Tauchbad oder durch Aufspritzen auf das Flachmaterial aufgebracht werden.

Für einen hohen Durchsatz ist es vorteilhaft, wenn das vorzugsweise durch Edelstahlblech gebildete Flachmaterial von Rolle zu Rolle durch photochemisches Maskenätzen verarbeitet wird. Denkbar ist es jedoch auch, einen Halbleiter-Wafer als Flachmaterial einzusetzen.

Vorteilhafterweise beträgt die Dicke des Flachmaterials zwischen 1 mm und 0,01 mm.

Gemäß einer weiteren vorteilhaften Ausgestaltung wird das Stechelement durch einen Kanalschlitz in der Maske mit einem halboffenen Kapillarkanal zum Transport der Körperflüssigkeit versehen, wobei die Kanalwandungen an ihrem distalen Ende als scharfe Spitze ausgebildet werden. Dabei ist es vorgesehen, dass die Abschirmbereiche für die Spitzen der Kanalwandungen durch den Kanalschlitz einseitig begrenzt werden.

Für eine besondere Formteilgestaltung wird das Flachmaterial beidseitig mit einer Maske versehen, wobei an der einen Seite ein spitzer Stechschaft und an der anderen Seite ein auf dem Stechschaft verlaufender halboffener Kapillarkanal mit spitzen Kanalwandungen freigeätzt wird.

Gemäß einem weiteren Erfindungsaspekt wird die Ätzmaske im seitlichen Abstand zu einem unterätzten Randabschnitt mit einer Kompensationsöffnung versehen, und es wird durch die Kompensationsöffnung hindurch unter Einwirkung des Ätzmittels eine Randkontur des Flachformteils kantenseitig abgeätzt. Auf diese Weise lassen sich unerwünschte Formteilhinterschnitte ohne zusätzlichen Fertigungsaufwand chemisch abfräsen.

Für eine Kantenbeseitigung ist es vorteilhaft, wenn die Kompensationsöffnung vorzugsweise als Schlitz oder Löcherkette in der Maske längs des Randabschnitts verläuft. Dabei sollte die Kompensationsöffnung eine geringere Öffnungsbreite als eine an den Randabschnitt angrenzende Maskenfreisparung aufweisen.

Um eine kombinierte Unterätzwirkung auf eine zwischen dem Maskenrand und der Kompensationsaussparung verlaufende Materialbrücke zu erzielen, sollte die Breite der darüberliegenden Maskenbrücke kleiner als die Unterätzweite des Ätzmittels von dem Maskenrand her sein.

In Abhängigkeit von dem gewünschten Konturverlauf ist es vorgesehen, dass die Kompensationsöffnung in einem Formbereich und gegebenenfalls einem Abschirmbereich der Maske für die zu bildende Spitze angeordnet ist. Speziell ist es günstig, wenn die Kompensationsöffnung im seitlichen Abstand zu einer abgewinkelten oder konkav gekrümmten Seitenflanke der Maske angeordnet ist.

Um auch in diesem Fall die Spitze gegen frontales Abätzen zu schützen, ist es von besonderem Vorteil, wenn die Kompensationsöffnung seitlich von einer in Richtung der zu bildenden Spitze verlaufenden Zentralachse eingebracht wird, so dass ein Maskenstreifen entlang der Zentralachse zumindest über die Unterätzweite hinaus vor der Spitze erhalten bleibt. Prinzipiell gelten hier dieselben Überlegungen hinsichtlich der Vermeidung einer frontalen Einwirkung des Ätzmittels wie vorstehend zu dem Maskenrand ausgeführt. In jedem Fall sollte also eine V-förmige Kontur der Kompensationsöffnung vermieden werden.

Im Folgenden wird die Erfindung anhand der in der Zeichnung schematisch dargestellten Ausführungsbeispiele näher erläutert. Es zeigen:
- Fig. 1: ein Stechelement mit einer Spitze und einem Kapillarka- nal zur Blutentnahme in der Draufsicht;
- Fig. 2: eine rückseitige Ätzmaske zur Herstellung der Spitze des Stechelements in der Draufsicht;
- Fig. 3: eine vorderseitige Ätzmaske zur Herstellung des Kapil- larkanals im Bereich der Spitze in der Draufsicht;
- Fig. 4: einen vergrößerten Ausschnitt der Ätzmaske im distalen Endbereich des Kapillarkanals in der Draufsicht;
- Fig. 5: einen Schnitt entlang der Linie 5-5 der Fig. 4;
- Fig. 6: einen Schnitt längs der Linie 6-6 der Fig. 4;
- Fig. 7 bis 9: ein weiteres Ausführungsbeispiel mit einem Kompensa- tionsschlitz in der Ätzmaske in Fig. 4 bis 6 entsprechen- den Darstellungen; und
- Fig. 10: eine Draufsicht auf eine Ätzmaske zur Herstellung einer Spitze nach dem Stand der Technik.

Das in der Zeichnung dargestellte Stech- bzw. Probenentnahmeelement 10 dient als Einwegteil zur Entnahme und zum Kapillartransport einer kleinen Blutmenge aus einer Körperpartie eines Probanden insbesondere zur Durchführung von Blutglucosemessungen. Es umfasst hierzu ein Flachformteil 12, ein daran angeformtes Stechorgan 14 mit Spitze 16 und einen halboffenen Kapillarkanal 18 zum kapillaren Bluttransport von der Spitze 16 zu einer Analysestelle 20.

Das Flachformteil 12 ist aus Edelstahlblech 22 mit einer Dicke von etwa 100 bis 300 µm gebildet. Sein proximaler Endabschnitt bildet einen Haltebereich für die Handhabung beim Stechvorgang, während das am distalen Ende einstückig angeformte Stechorgan 14 in der Haut eines Benutzers eine kleine Wunde erzeugt, um mikroskopische Volumina an Blut entnehmen zu können. Der Kapillarkanal 18 ist dabei über seien Länge rillenförmig bzw. halboffen ausgebildet, so dass wie nachstehend beschrieben eine photolithographische Herstellung möglich ist. Die Analyse der entnommenen Blutprobe lässt sich beispielsweise durch reflexionsspektroskopische oder elektrochemische Nachweismethoden in an sich bekannter Weise realisieren.

Die Strukturierung des Blechmaterials bzw. Substrats erfolgt nach dem PCM-Verfahren (engl. "photochemical machining" oder "milling"). Hierbei wird das Substrat 22 vorzugsweise beidseitig mit einer Ätzmaske 24 versehen, welche die in einem nachfolgenden Ätzschritt freizulegende Formteilstruktur abdeckt. Zur Maskenbildung wird das Substrat 22 mit einem Photolack("photoresist") beschichtet und über eine vorgeordnete Photomaske mit dem gewünschten Muster belichtet, wobei der Photolack in den abgedeckten Bereichen polymerisiert bzw. gehärtet wird, während die übrigen Bereiche nach dem Entwickeln freigespült werden.

Über die so erzeugte (doppelseitige) Ätzmaske 24 wird anschließend das Substrat 22 mit einem Ätzmittel beaufschlagt, so dass die maskierten Bereiche der Grundform nach freigeätzt werden. Bei einer isotropen Ätzwirkung entspricht der Materialabtrag in die Tiefe der lateralen Ätzrate beim Unterätzen von Randkonturen der Maske 24. Durch äußere Einflussparameter bzw. Materialeigenschaften des Substrats kann der Ätzvorgang auch anisotrop erfolgen, d. h. die laterale Unterätzrate ist dann größer oder kleiner als die Tiefenätzrate.

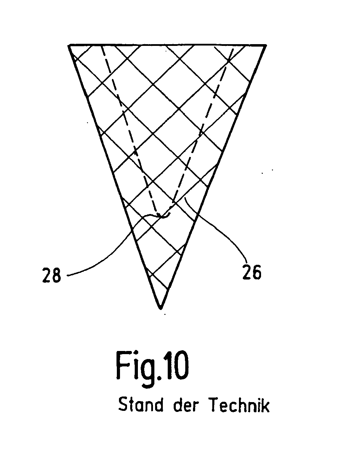

Besonders kritisch für die Funktion des Stechelements 10 ist die Herstellung der Spitze 16. Gemäß Fig. 10 besteht ein naheliegender Ansatz nach dem Stand der Technik darin, eine entsprechende Ätzmaske mit einem spitz zulaufenden bzw. dreieckförmigen Formbereich 26 entsprechend der gewünschten Kontur des Fertigteils vorzusehen. Hierbei wurde jedoch beobachtet, dass die so gebildete Spitze 28 nicht scharf ist, sondern aufgrund des allseitig anflutenden Ätzmittels beim Unterätzen der Dreieckmaske 26 abgerundet wird.

Um dies zu vermeiden, weist die erfindungsgemäße Ätzmaske 24 einen Formbereich 30 für die zu bildende Spitze 16 und einen distal -in Stechrichtung gesehen - daran anschließenden Abschirmbereich 32 gegen frontales Abätzen der Spitze 16 auf. Der Formbereich 30 verengt sich zu dem Abschirmbereich 32 hin, wobei die Seitenflanken 34, 36 der Maske 24 mit linearer Steigung abgeschrägt sind. Ausgehend von einer Engstelle 38 erweitert sich der Abschirmbereich 32 unter Bildung eines Brückenabschnitts 40 zu den übrigen Maskenbereichen hin, so dass die Ätzmaske 24 insgesamt stabiler bleibt.

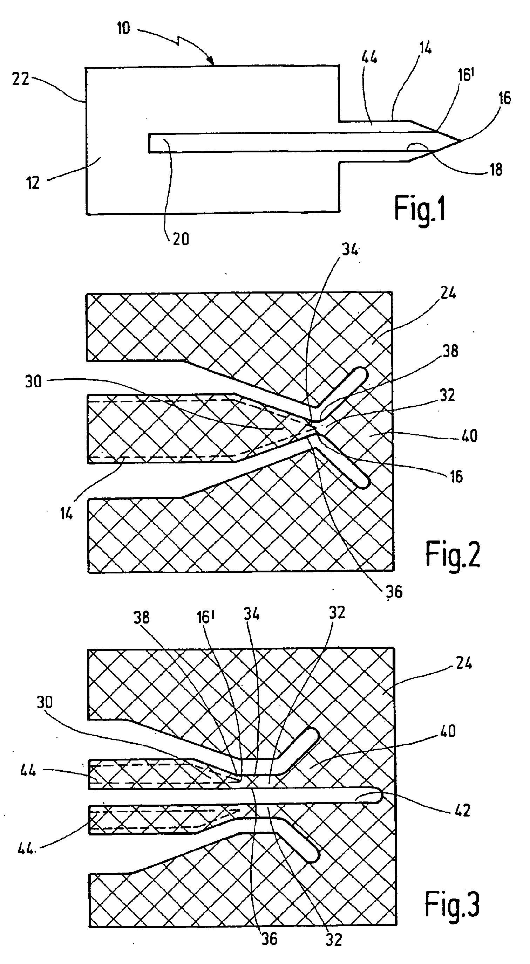

Durch laterales Unterätzen der Seitenflanken 34, 36 des Form- und Abschirmbereichs 30, 32 wird somit eine scharfe Spitze 16 freigeätzt, deren Kontur in Fig. 2 gestrichelt dargestellt ist. Dabei besitzt der Abschirmbereich 32 eine größere Abschirmlänge als die Unterätzweite in proximaler Richtung gesehen, während die Breite der Engstelle 38 kleiner als die doppelte laterale Unterätzweite ist. Auf diese Weise laufen die abgeätzten Materialfronten an der Engstelle 38 aufeinander zu, bis schließlich die Spitze 16 beim Beenden des Ätzprozesses freigelegt wird.

Der in Fig. 3 gezeigte vorderseitige Ausschnitt der Maske 24 ist komplementär zu der gewünschten Kapillarstruktur 18 im Bereich des Stechorgans 14 gestaltet. Demgemäß weist die Maske 24 einen Kapillarschlitz 42 auf, durch welchen der Kanal 18 unter Bildung der Kanalwandungen 44 eingeätzt wird. Um auch hier den Einstich zu erleichtern, werden die Kanalwandungen 44 an ihren distalen Enden als scharfe Spitzen 16' abgeschrägt. Dies wird in oben beschriebener Weise durch vorgelagerte Abschirmbereiche 32 erreicht, wobei in Fig. 3 funktionell gleiche Teile mit gleichen Bezugszeichen versehen sind, so dass auf vorstehende Ausführungen verwiesen werden kann. Im Unterschied zu Fig. 2 sind die Seitenflanken 34, 36 im Bereich der Engstelle 38 nicht beidseitig konkav gekrümmt, sondern einseitig stumpf abgewinkelt und gegenüberliegend durch den Kapillarspalt 42 geradlinig begrenzt, so dass eine keilförmige Spitze 16' wie gestrichelt dargestellt gebildet wird.

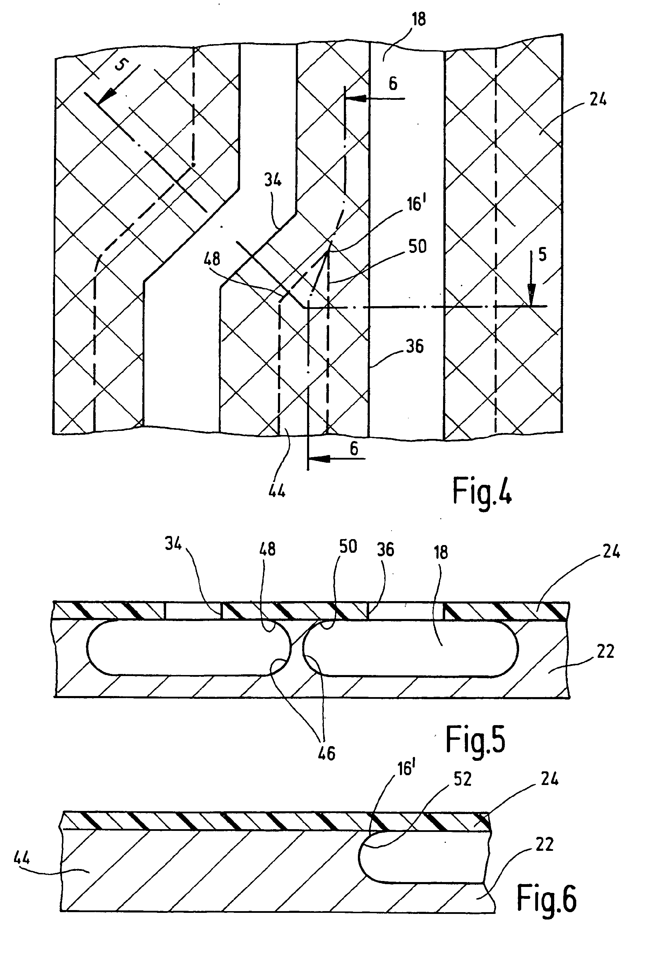

In den Fig. 4 bis 6 sind die prinzipiellen geometrischen Auswirkungen des Ätzprozesses im Bereich der Kanalspitzen 16' veranschaulicht. Die Fig. 5, 6 zeigen dabei nur den oberen Substratbereich nach einer gewissen Ätzzeit, wobei die seitlichen Ätzkonturen 46 nur bei isotroper Ätzwirkung eine Kreislinie beschreiben. Durch laterales Unterätzen der Maskenränder bzw. Seitenflanken 34, 36 der Maske 24 ergeben sich somit hinterschnittene Formteilkanten 48, 50, wie am besten aus Fig. 5 ersichtlich. Solche Hinterschnitte sind im Bereich des Kapillarkanals 18 durchaus erwünscht, weil dadurch die Kapillarität weiter verbessert wird. An der Spitze 16, 16' ergibt sich jedoch durch den Hinterschnitt 52 ein Widerhaken (Fig. 6), welcher den Einstich in die Haut beeinträchtigen kann.

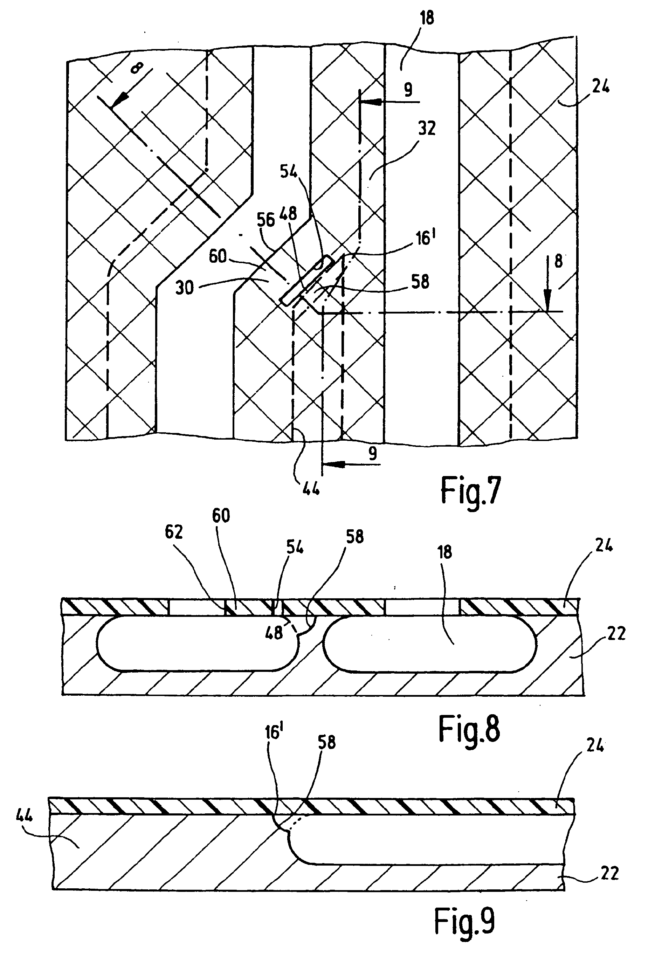

Um hier Abhilfe zu schaffen, kann in der Maske 24 gemäß Fig. 7 und 8 ein Kompensationsschlitz 54 freigehalten werden. Dieser verläuft im seitlichen Abstand zu einem unterätzen Randabschnitt 56 im Formbereich 30 der Maske 24 und sorgt dafür, dass der ansonsten entstehende Hinterschnitt 48 kantenseitig abgeätzt wird. Das über den Kompensationsschlitz 54 kantennahe eindringende Ätzmittel führt somit zu einer Kantenabrundung 58 unter Vermeidung eines Widerhakens. Zweckmäßig ist die Breite des Maskenstreifens 60 zwischen dem Maskenrand 56 und dem Kompensationsschlitz 54 kleiner als die laterale Unterätzweite. Dabei sollte gewährleistet sein, dass der Kompensationsschlitz 54 im Vergleich zu der benachbarten Maskenfreisparung 62 eine wesentlich geringere Öffnungsbreite besitzt, so dass die Kantenabrundung 58 einen entsprechend kleineren Ätzradius aufweist.

## Patentansprüche

1. Verfahren zur Herstellung eines Stechelements insbesondere zur Entnahme einer Körperflüssigkeit, bei welchem aus einem Flachmaterial (22) unter Aufbringen einer Maske (24) und Einwirkung eines Ätzmittels ein Flachformteil (12) gebildet wird, wobei mindestens eine Partie des Flachformteils (12) als Spitze (16,16') zum Einstechen in ein Körperteil eines Probanden ausgeformt wird, wobei die Maske (24) mit einem Formbereich (30) für die zu bildende Spitze (16,16') und einem distal darüber hinaus sich erstreckenden Abschirmbereich (32) gegen frontales Abätzen der Spitze (16,16') versehen wird, **dadurch gekennzeichnet, dass** zumindest eine Seitenflanke (34) im Übergang zwischen Formbereich (30) und Abschirmbereich (32) abgewinkelt oder konkav gekrümmt ist, so dass der Abschirmbereich (32) über eine gegebene Abschirmlänge im Anschluss an den Formbereich (30) eine gleich bleibende oder zunehmende Breite aufweist und nur durch laterales Unterätzen der Seitenflanken (34,36) des Form- und Abschirmbereichs (32) eine scharfe Spitze (16,16') freigelegt wird.

2. Verfahren nach Anspruch 1, **dadurch gekennzeichnet, dass** der Formbereich (30) sich zu dem Abschirmbereich (32) hin verengt.

3. Verfahren nach Anspruch 1 oder 2, **dadurch gekennzeichnet, dass** der Formbereich (30) zumindest einseitig linear abgeschrägt ist.

4. Verfahren nach Anspruch 1, **dadurch gekennzeichnet, dass** die Abschirmlänge größer als die proximale Unterätzweite aufgrund der Einwirkung des Ätzmittels ist.

5. Verfahren nach einem der Ansprüche 1 bis 4, **dadurch gekennzeichnet, dass** die Spitze (16,16') durch eine Engstelle (38) der Maske (24) geformt wird.

6. Verfahren nach Anspruch 5, **dadurch gekennzeichnet, dass** die Breite der Engstelle (38) kleiner als die zweifache laterale Unterätzweite ist.

7. Verfahren nach Anspruch 5 oder 6, **dadurch gekennzeichnet, dass** der Abschirmbereich (32) im Anschluss an die Engstelle (38) wieder verbreitert wird.

8. Verfahren nach einem der Ansprüche 1 bis 7, **dadurch gekennzeichnet, dass** der Abschirmbereich (32) einen in distaler Richtung der zu bildenden Spitze (16,16') gesehen sich erweiternden Brückenabschnitt (40) aufweist.

9. Verfahren nach einem der Ansprüche 1 bis 8, **dadurch gekennzeichnet, dass** die laterale Unterätzweite größer oder kleiner als die Ätztiefe ist.

10. Verfahren nach einem der Ansprüche 1 bis 9, **dadurch gekennzeichnet, dass** das Ätzmittel im Tauchbad oder durch Aufspritzen auf das Flachmaterial aufgebracht wird.

11. Verfahren nach einem der Ansprüche 1 bis 10, **dadurch gekennzeichnet, dass** das Ätzmittel unter Überdruck gegenüber der Umgebung angewandt wird.

12. Verfahren nach einem der Ansprüche 1 bis 11, **dadurch gekennzeichnet, dass** das vorzugsweise durch Edelstahlblech gebildete Flachmaterial (22) von Rolle zu Rolle durch photochemisches Maskenätzen verarbeitet wird.

13. Verfahren nach einem der Ansprüche 1 bis 12, **dadurch gekennzeichnet, dass** die Dicke des Flachmaterials (22) zwischen 1 mm und 0,01 mm gewählt wird.

14. Verfahren nach einem der Ansprüche 1 bis 13, **dadurch gekennzeichnet, dass** das Stechelement durch einen Kanalschlitz (42) in der Maske (24) mit einem halboffenen Kapillarkanal (18) zum Transport der Körperflüssigkeit versehen wird, und dass die Kanalwandungen (44) an ihrem distalen Ende als scharfe Spitze (16') ausgebildet werden.

15. Verfahren nach Anspruch 14, **dadurch gekennzeichnet, dass** die Abschirmbereiche (32) für die Spitzen (16') der Kanalwandungen (44) durch den Kanalschlitz (42) einseitig begrenzt werden.

16. Verfahren nach einem der Ansprüche 1 bis 15, **dadurch gekennzeichnet, dass** das Flachmaterial (22) beidseitig mit einer Maske (24) versehen wird, wobei an der einen Seite ein spitzer Stechschaft (14) und an der anderen Seite ein auf dem Stechschaft (14) verlaufender halboffener Kapillarkanal (18) mit spitzen Kanalwandungen (44) freigeätzt wird.

17. Verfahren nach einem der Ansprüche 1 bis 16, **dadurch gekennzeichnet, dass** die Maske (24) im seitlichen Abstand zu einem unterätzten Randabschnitt (56) mit einer Kompensationsöffnung (54) versehen wird, und dass durch die Kompensationsöffnung (54) unter Einwirkung des Ätzmittels eine Randkontur (48) des Flachformteils (12) kantenseitig abgeätzt wird.

18. Verfahren zur Herstellung eines Stechelements insbesondere zur Entnahme einer Körperflüssigkeit, bei welchem aus einem Flachmaterial (22) unter Aufbringen einer Maske (24) und Einwirkung eines Ätzmittels ein Flachformteil (12) gebildet wird, wobei mindestens eine Partie des Flachformteils (12) als Spitze (16,16') zum Einstechen in ein Körperteil eines Probanden ausgeformt wird, wobei die Maske (24) im seitlichen Abstand zu einem unterätzten Randabschnitt (56) mit einer Kompensationsöffnung (54) versehen wird, und durch die Kompensationsöffnung (54) unter Einwirkung des Ätzmittels eine Randkontur (48) des Flachformteils (12) kantenseitig abgeätzt wird, **dadurch gekennzeichnet, dass** die Kompensationsöffnung (54) nur seitlich von einer in Richtung der zu bildenden Spitze (16,16') verlaufenden Zentralachse eingebracht wird, so dass ein Maskenstreifen entlang der Zentralachse vor der Spitze (16,16') erhalten bleibt und die Spitze (16,16') gegen frontales Abätzen abgeschirmt wird.

19. Verfahren nach Anspruch 17 oder 18, **dadurch gekennzeichnet, dass** die Kompensationsöffnung (54) vorzugsweise als Schlitz oder Löcherkette in der Maske (24) längs des Randabschnitts (56) verläuft.

20. Verfahren nach einem der Ansprüche 17 bis 19, **dadurch gekennzeichnet, dass** die Kompensationsöffnung (54) eine geringere Öffnungsbreite als eine an den Randabschnitt (56) angrenzende Maskenfreisparung (62) aufweist.

21. Verfahren nach einem der Ansprüche 17 bis 20, **dadurch gekennzeichnet, dass** zwischen dem Maskenrand und der Kompensationsöffnung (54) eine Maskenbrücke (60) verläuft, und dass die Breite der Maskenbrücke (60) kleiner als die Unterätzweite des Ätzmittels von dem Maskenrand her ist.

22. Verfahren nach einem der Ansprüche 17 bis 21, **dadurch gekennzeichnet, dass** die Kompensationsöffnung (54) in einem Formbereich (30) und gegebenenfalls einem Abschirmbereich (32) der Maske (24) für die zu bildende Spitze (16,16') angeordnet wird.

23. Verfahren nach einem der Ansprüche 17 bis 22, **dadurch gekennzeichnet, dass** die Kompensationsöffnung (54) im seitlichen Abstand zu einer über die zu bildende Spitze (16,16') distal hinaus abgewinkelten oder konkav gekrümmten Seitenflanke (34) der Maske (24) angeordnet wird.

## Claims

1. Process for producing a lancing element in particular for withdrawing a body fluid in which a flat shaped member (12) is formed from a flat material (22) by applying a mask (24) and allowing an etching agent to act upon it wherein at least a part of the flat shaped member (12) is shaped as a tip (16, 16') for piercing into a body part of a test subject, wherein the mask (24) is provided with a shaping area (30) for the tip to be shaped (16, 16') and with a screening area (32) which extends distally beyond this shaping area to prevent the tip from being etched off at the front (16, 16'), **characterized in that** at least one side flank (34) is bent or concavely curved in the junction between the shaping area (30) and screening area (32) so that the screening area (32) has a constant or increasing width over a given screening length where it connects with the shaping area (30) and a sharp tip (16, 16') is exposed only by laterally undercutting the side flanks (34, 36) of the shaping area and screening area (32).

2. Process according to claim 1, **characterized in that** the shaping area (30) of the etching mask narrows towards the screening area (32).

3. Process according to claim 1 or 2, **characterized in that** the shaping area (30) is linearly chamfered at least on one side.

4. Process according to claim 1, **characterized in that** the screening length is larger than the proximal undercutting width due to the action of the etching agent.

5. Process according to one of the claims 1 to 4, **characterized in that** the tip (16, 16') is shaped by a constriction (38) in the mask (24).

6. Process according to claim 5, **characterized in that** the width of the constriction (38) should be less than twice the lateral undercutting width.

7. Process according to claim or 6, **characterized in that** the screening area (32) is widened again after the constriction (38).

8. Process according to one of the claims 1 to 7, **characterized in that** the screening area (32) has a bridge section (40) that widens in the distal direction relative to the tip (16, 16') that is to be formed.

9. Process according to one of the claims 1 to 8, **characterized in that** the undercutting etching width is larger or smaller than the etching depth.

10. Process according to one of the claims 1 to 9, **characterized in that** the etching agent is applied in a dipping bath or by being sprayed onto the flat material.

11. Process according to one of the claims 1 to 10, **characterized in that** the etching agent is applied at an overpressure relative to the surroundings.

12. Process according to one of the claims 1 to 11, **characterized in that** the flat material (22) that is preferably formed from stainless steel sheet metal is processed from roll to roll by photochemical mask etching.

13. Process according to one of the claims 1 to 12, **characterized in that** the thickness of the flat material (22) is selected to be between I mm and 0.01 mm.

14. Process according to one of the claims 1 to 13, **characterized in that** the lancing element is provided with a semi-open capillary channel (18) to transport the body fluid that is formed by a channel slot (42) in the mask (24), and that the distal ends of the channel walls (44) are shaped as a sharp tip (16').

15. Process according to claim 14, **characterized in that** the screening areas (32) for the tips (16') of the channel walls (44) are delimited on one side by the channel slot (42).

16. Process according to one of the claims 1 to 15, **characterized in that** the flat material (22) is provided on both sides with a mask (24) wherein a pointed lancing shaft (14) is etched free on one side and a semi-open capillary channel (18) with pointed channel walls (44) that extends towards the lancing shaft (14) is etched free on the other side.

17. Process according to one of the claims 1 to 16, **characterized in that** the mask (24) is provided with a compensation opening (54) at a lateral distance from an undercut edge section (56), and that an edge contour (48) of the flat shaped member (12) is etched away from the edge through the compensation opening (54) under the action of the etching agent.

18. Process for producing a lancing element in particular for withdrawing a body fluid in which a flat shaped member (12) is formed from a flat material (22) by applying a mask (24) and allowing an etching agent to act upon it wherein at least a part of the flat shaped member (12) is shaped as a tip (16, 16') for piercing into a body part of a test subject, wherein the mask (24) is provided with a compensation opening (54) at a lateral distance from an undercut edge section (56), and an edge contour (48) of the flat shaped member (12) is etched away from the edge through the compensation opening (54) under the action of the etching agent, **characterized in that** the compensation opening (54) is positioned only at the side of a central axis running towards the tip (16, 16') that is to be shaped so that a strip of the mask is retained in front of the tip (16, 16') along the central axis and the tip (16, 16') is screened from being etched off at the front.

19. Process according to claim 17 or 18, **characterized in that** the compensation opening (54) runs preferably as a slot or chain of holes in the mask (24) along the edge section (56).

20. Process according to one of the claims 17 to 19, **characterized in that** the compensation opening (54) has a smaller inner width than a cut-away in the mask (62) adjoining the edge section (56).

21. Process according to one of the claims 17 to 20, **characterized in that** a mask bridge (60) runs between the mask edge and the compensation opening (54), and that the width of the mask bridge (60) is less than the undercutting width of the etching agent measured form the edge of the mask.

22. Process according to one of the claims 17 to 21, **characterized in that** the compensation opening (54) is arranged in a shaping area (30) and optionally a screening area (32) of the mask (24) for the tip (16, 16') to be shaped.

23. Process according to one of the claims 17 to 22, **characterized in that** the compensation opening (54) is laterally spaced apart from a side flank (34) of the mask (24) that is bent or concavely curved distally beyond the tip (16, 16') to be shaped.

## Revendications

1. Procédé de fabrication d'un élément de piquage notamment destiné au prélèvement d'un liquide organique, dans lequel on forme à partir d'un matériel plat (22) par application d'un masque (24) et sous l'effet d'un agent de gravure une pièce moulée plate (12), au moins une partie de ladite pièce moulée plate (12) étant formée en pointe (16, 16') pour piquer une partie du corps d'un sujet à examiner, ledit masque (24) étant pourvu d'une zone de formage (30) pour la pointe à former (16, 16') et d'une zone de protection (32) s'étendant distalement au-delà de ladite zone de formage pour protéger la pointe (16, 16') contre une attaque chimique frontale, **caractérisé en ce qu'**au moins un flanc latéral (34) situé dans la transition entre zone de formage (30) et zone de protection (32) est incliné ou présente une courbure concave de sorte que la zone de protection (32) présente sur une longueur de protection donnée à la suite de la zone de formage (30) une largeur constante ou croissante et qu'une pointe acérée (16, 16') n'est dégagée que par une sous-gravure latérale des flancs latéraux (34, 36) de la zone de formage et de protection (32).

2. Procédé selon la revendication 1, **caractérisé en ce que** la zone de formage (30) se resserre vers la zone de protection (32).

3. Procédé selon la revendication 1 ou 2, **caractérisé en ce que** la zone de formage (30) est biseautée linéairement au moins d'un côté.

4. Procédé selon la revendication 1, **caractérisé en ce que** la longueur de protection est supérieure à la largeur de sous-gravure proximale en raison de l'effet de l'agent de gravure.

5. Procédé selon l'une des revendications 1 à 4, **caractérisé en ce que** la pointe (16, 16') est formée par une constriction (38) du masque (24).

6. Procédé selon la revendication 5, **caractérisé en ce que** la largeur de ladite constriction (38) est inférieure à deux fois la largeur de sous-gravure latérale.

7. Procédé selon la revendication 5 ou 6, **caractérisé en ce que** la zone de protection (32) à la suite de ladite constriction (38) redevient plus large.

8. Procédé selon l'une des revendications 1 à 7, **caractérisé en ce que** la zone de protection (32) présente une partie en pont évasée (40), vu dans le sens distal de la pointe à former (16, 16').

9. Procédé selon l'une des revendications 1 à 8, **caractérisé en ce que** la largeur de sous-gravure latérale est supérieure ou inférieure à la profondeur de gravure.

10. Procédé selon l'une des revendications 1 à 9, **caractérisé en ce que** l'agent de gravure est appliqué par immersion ou au pistolet sur le matériel plat.

11. Procédé selon l'une des revendications 1 à 10, **caractérisé en ce que** l'agent de gravure est utilisé en surpression par rapport au milieu environnant.

12. Procédé selon l'une des revendications 1 à 11, **caractérisé en ce que** le matériel plat (22), constitué de préférence par une tôle d'acier inoxydable, est usiné, bobine par bobine, par gravure photochimique d'un masque.

13. Procédé selon l'une des revendications 1 à 12, **caractérisé en ce que** l'épaisseur du matériel plat (22) est choisie entre 1 mm et 0,01 mm.

14. Procédé selon l'une des revendications 1 à 13, **caractérisé en ce que** l'élément de piquage, à travers une fente de canal (42) ménagée dans le masque (24), est muni d'un canal capillaire semi-ouvert (18) destiné au transport du liquide organique, et **en ce que** les parois de canal (44), à leur extrémité distale, sont réalisées sous forme de pointe acérée (16').

15. Procédé selon la revendication 14, **caractérisé en ce que** les zones de protection (32) pour les pointes (16') des parois de canal (44) sont limitées sur un côté par ladite fente de canal (42).

16. Procédé selon l'une des revendications 1 à 15, **caractérisé en ce que** le matériel plat (22) est muni des deux côtés d'un masque (24), sur l'un des côtés étant dégagée par attaque chimique une tige de piqûre pointue (14) et de l'autre côté étant dégagé un canal capillaire semi-ouvert (18) s'étendant sur ladite tige de piqûre (14), avec des parois de canal pointues (44).

17. Procédé selon l'une des revendications 1 à 16, **caractérisé en ce que** le masque (24), à distance latérale par rapport à une partie périphérique sous-gravée (56), est muni d'une ouverture de compensation (54), et **en ce qu'**à travers ladite ouverture de compensation (54), sous l'effet de l'agent de gravure, un contour périphérique (48) de la pièce moulée plate (12) est éliminé par attaque chimique du côté du bord.

18. Procédé de fabrication d'un élément de piquage notamment destiné au prélèvement d'un liquide organique, dans lequel on forme à partir d'un matériel plat (22) par application d'un masque (24) et sous l'effet d'un agent de gravure une pièce moulée plate (12), au moins une partie de ladite pièce moulée plate (12) étant formée en pointe (16, 16') pour piquer une partie du corps d'un sujet à examiner, ledit masque (24), à distance latérale par rapport à une partie périphérique sous-gravée (56), étant muni d'une ouverture de compensation (54), et à travers ladite ouverture de compensation (54), sous l'effet de l'agent de gravure, un contour périphérique (48) de la pièce moulée plate (12) étant éliminé par attaque chimique du côté du bord, **caractérisé en ce que** ladite ouverture de compensation (54) est ménagée uniquement sur le côté d'un axe central s'étendant en direction de la pointe à former (16, 16') de sorte qu'une bande de masque est préservée le long de l'axe central avant la pointe (16, 16'), protégeant ladite pointe (16, 16') contre une attaque chimique frontale.

19. Procédé selon la revendication 17 ou 18, **caractérisé en ce que** l'ouverture de compensation (54) s'étend de préférence sous forme de fente ou chaîne à trous dans le masque (24) le long de la partie périphérique (56).

20. Procédé selon l'une des revendications 17 à 19, **caractérisé en ce que** l'ouverture de compensation (54) présente une moindre largeur d'ouverture qu'un évidement de masque (62) contigu à la partie périphérique (56).

21. Procédé selon l'une des revendications 17 à 20, **caractérisé en ce qu'**un pont de masque (60) s'étend entre le bord de masque et l'ouverture de compensation (54), et **en ce que** la largeur dudit pont de masque (60) est inférieure à la largeur de sous-gravure de l'agent de gravure depuis le bord de masque.

22. Procédé selon l'une des revendications 17 à 21, **caractérisé en ce que** l'ouverture de compensation (54) est agencée dans une zone de formage (30) et éventuellement une zone de protection (32) du masque (24) pour la pointe à former (16, 16').

23. Procédé selon l'une des revendications 17 à 22, **caractérisé en ce que** l'ouverture de compensation (54) est agencée à distance latérale par rapport à un flanc latéral (34) incliné ou présentant une courbure concave du masque (24), qui s'étend distalement au-delà de la pointe à former (16, 16').
